(19) **Europäisches Patentamt / European Patent Office / Office européen des brevets**

(11) **EP 1 698 264 B1**

(12) **EUROPEAN PATENT SPECIFICATION**

(45) Date of publication and mention
of the grant of the patent:
**22.06.2016 Bulletin 2016/25**

(51) Int Cl.:
*A61B 1/00* (2006.01)    *A61B 5/06* (2006.01)
*A61B 5/07* (2006.01)

(21) Application number: **04788429.1**

(22) Date of filing: **30.09.2004**

(86) International application number:
**PCT/JP2004/014401**

(87) International publication number:
**WO 2005/065521 (21.07.2005 Gazette 2005/29)**

(54) **SYSTEM FOR SENSING MOVEMENT IN SUBJECT**

SYSTEM ZUM NACHWEIS VON BEWEGUNGEN IN EINER PERSON

SYSTEME DESTINE A DETECTER UN MOUVEMENT DANS LE CORPS D'UN SUJET

(84) Designated Contracting States:
**DE FR GB**

(30) Priority: **26.12.2003 JP 2003435557**

(43) Date of publication of application:
**06.09.2006 Bulletin 2006/36**

(73) Proprietor: **Olympus Corporation**
**Tokyo 151-0072 (JP)**

(72) Inventors:
• **MINAI, Tetsuo;**
**c/o OLYMPUS CORPORATION**
**Tokyo 1920023 (JP)**
• **SHIMIZU, Hatsuo**
**Tokyo 1920024 (JP)**

(74) Representative: **von Hellfeld, Axel**
**Wuesthoff & Wuesthoff**
**Patentanwälte PartG mbB**
**Schweigerstrasse 2**
**81541 München (DE)**

(56) References cited:
WO-A-00/27281          WO-A-01/87377
JP-A- 4 008 341        JP-A- 9 028 662
JP-A- 2003 019 111     JP-A- 2003 117 004
US-A1- 2002 198 439    US-A1- 2002 198 470
US-A1- 2003 073 935    US-A1- 2003 114 742

• WEITSCHIES WERNER: "Magnetic marker monitoring of disintegrating capsules" EUROPEAN JOURNAL OF PHARMACEUTICAL SCIENCES, vol. 13, 2001, pages 411-416, XP002535579

**Description**

TECHNICAL FIELD

The present invention relates to a system for detecting a travel state of an body-insertable device in a body of a subject, said system including the device, which once being swallowed, travels naturally autonomously through the body of the subject, and a travel state detector that is disposed on the outside of the body of the subject and obtains information on the current position of the body-insertable device.

BACKGROUND ART

In recent years, in the field of endoscopes, a swallowable capsule endoscope has been proposed. The capsule endoscope has an image capturing function and a radio communication function. The capsule endoscope has the function of traveling in the body cavity of the subject, in the organs such as the stomach and the small intestine with peristalsis of the organs and sequentially capturing images for a period of time since the capsule endoscope is swallowed from the mouth of the subject for inspection (examination) until it is naturally excreted.

Image data captured in the body of the subject by the capsule endoscope as the capsule endoscope travels in the body cavity is sequentially transmitted by the radio communication function out from the body of the subject and stored into memory externally insert or removed. By carrying a receiver having a radio communication function and a storing function, the subject can freely move throughout the period after he/she swallows the capsule endoscope until it is excreted. After the capsule endoscope is excreted, a doctor or nurse can display the images of the organs reproduced based on the image data stored in the memory on a display to make an examination.

A capsule endoscope system has been proposed, in which the receiver has the function of detecting the current position of the capsule endoscope in the body of the subject to capture, for example, an endoscope image of a specific organ thereof. As an example of the capsule endoscope system having the position detecting function, a capsule endoscope system using the radio communication function provided in the capsule endoscope is known. Specifically, the system has a configuration that a receiver provided on the outside of a subject (externally installed) has a plurality of antenna elements for receiving radio signals transmitted from the capsule endoscope and a position detecting function for detecting the current position of the capsule endoscope in the subject based on a difference among the intensities of the signals received by the antenna elements (see Patent Document 1, for example).

It is also possible to determine the travel state of the capsule endoscope using a position detecting mechanism. For example, the travel speed can be determined based on a positional change in a predetermined time. The intervals of image pick-up inside the subject can be adjusted based on the travel speed of the capsule endoscope. In other words, images of inside the subject can be acquired at regular distance intervals, regardless of the travel speed of the capsule endoscope, by decreasing the image pick-up interval in a region where the travel speed of the capsule endoscope is high and increasing the image pick-up interval in a region where the travel speed is low.

Patent Document 1: Japanese Patent Application Laid-open No. 2003-19111

Document US 2003/0114742 A1 discloses a system for controlling a capsule in vivo. A magnetic field generator, a processing and control unit and a position monitoring system are attached to a garment worn by a patient. The processing and control unit is input with positional data from the position monitoring system or is controlled by an external operator to guide the capsule swallowed by the patient to a desired location within the patient's body. Thereby, the processing and control unit sends a command signal to the magnetic field generator which then transmits a signal of an amplitude and vector to move or rotate the capsule. The capsule includes an imaging device with an illumination unit, an image sensor, a transmitter, a steerable transceiver and a power source.

Document WO00/27281 discloses a system for determining the location and the orientation of an indwelling medical device via magnetic sensors. An arbitrarily selected sensor is used as origin of the reference frame used for the calculation of the location and the orientation of the medical device

DISCLOSURE OF INVENTION

PROBLEM TO BE SOLVED BY THE INVENTION

However, a conventional capsule endoscopic system has a problem that the accuracy of position detection of the capsule endoscope within the subject is low. As below, such a problem will be described in detail.

The capsule endoscopic system according to the conventional technology performs position detection of the capsule endoscope within the subject based on the distribution of intensities received at the plural antenna elements provided in the receiver as described above. Such a position detection mechanism is performed on the assumption that the intensity attenuation of radio signal transmitted from the capsule endoscope is uniquely determined depending on the distance from the capsule endoscope as described in paragraph [0018] in Publication of Patent Application Laid-Open No. 2003-19111.

However, since objects such as organs existing between the capsule endoscope and the antenna elements have different relative permittivities and conductivities, the attenuation rates of radio signal intensity vary depending on the types of the interposing objects, for example.

When a liver, blood vessels, or the like exist between the capsule endoscope and the antenna elements, the organs absorb a large amount of radio signals, increasing the attenuation rate of the radio signal intensity compared to the case where such organs do not exist, and the accurate position detection is hindered.

Naturally, this problem also has adverse effect on the detection of the travel state of the capsule endoscope. Thus, if the travel state of the capsule endoscope is detected based on the inaccurate result of position detection, it is very difficult to obtain accurate information on the travel state of the capsule endoscope.

An object of the present invention, developed to solve the problems described _ above, is to provide a system for detecting a travel state of a capsule endoscope in the body of the subject, which is capable of accurately detecting the current position and travel state of an body-insertable device in the body of the subject, for example, a capsule endoscope, regardless of the presence of structures such as organs.

MEANS FOR SOLVING PROBLEM

The present invention provides a system for detecting a travel state in a body of a subject according to claim 1.

To solve the problems described above and to achieve an object, a system for detecting a travel state in a body of a subject includes an body-insertable device that, once being swallowed, travels through a body of the subject, and a travel state detector for detecting the travel state of the body-insertable device in the body of the subject. The body-insertable device includes a magnetic field generator for outputting a constant magnetic field to the outside of the body of the subject. The travel state detector includes at least a magnetic field detector that is disposed in a fixed position relative to the subject and detects an intensity of a constant magnetic field output from the magnetic field generator; a position calculator that calculates a position of the device in the subject based on the intensity of the magnetic field detected by the magnetic field detector; and a travel state processor that determines a travel state of the device in the body of the subject based on the position calculated by the position calculator.

According to the present invention, an body-insertable device has a magnetic field generator for forming a constant magnetic field, wherein based on the intensity of the constant magnetic field generated by the magnetic field generator, a travel state detector detects the position of the body-insertable device. Since the constant magnetic field generated by the magnetic field generator has a property that it linearly attenuates according to a distance away from the magnetic field generator, regardless of factors such as dielectric constant and permeability, the correct position of the body-insertable device may be detected, enabling the correct travel state to be calculated based on the determined correct position.

EFFECT OF THE INVENTION

According to the present invention, the body-insertable device has a the magnetic field generator for generating the constant magnetic field and the travel state detector detects a position of the body-insertable device in the body of the subject based on the intensity of the constant magnetic field generated by the magnetic field generator. Since the constant magnetic field has a property that it attenuates linearly according to the distance the magnetic field generator, regardless of the dielectric constant and permeability of structures in the body of the subject, thereby enabling correct detection of the position of the body-insertable device. The correct information on the travel state of the body-insertable device can be advantageously acquired based on the correct position information.

BRIEF DESCRIPTION OF DRAWINGS

FIG. 1 is a schematic view of a system for detecting a travel state of a capsule endoscope in a body of a subject according to an embodiment;
FIG. 2 is a schematic view of a capsule endoscope as a component of the system according to the embodiment;
FIG. 3 is a schematic view of a travel state processor as a component of the system according to the embodiment;
FIG. 4 is a flowchart of the principle operation of the travel state processor to calculate the position of the capsule endoscope;
FIG. 5 is a schematic view of the fundamental principle for calculating the position of a capsule endoscope by the travel state processor;

FIG. 6 is a flowchart of the principle operation of the travel state processor to derive an orientation direction of the capsule endoscope;

FIG. 7 is a schematic view of the fundamental principle for deriving the orientation direction of the capsule endoscope by the travel state processor;

FIG. 8 is a flowchart of the principle operation of determining a travel state of the capsule endoscope by a travel state information generator;

FIG. 9 is a schematic view of the test capsule as a component of the system according to a modification; and

FIG. 10 is a schematic view of the configuration of the travel state processor as a component of the system according to the modification.

EXPLANATIONS OF LETTERS OR NUMERALS

| 1 | subject |
| 2 | capsule endoscope |
| 3 | travel state detector |
| 4 | display |
| 5 | portable recording medium |
| 6a-6h | magnetic field detectors |
| 7a, 7b | fixing member |
| 8 | travel state processor |
| 9 | receiving antenna |
| 10 | transmitting antenna |
| 11 | LED |
| 12 | LED driving circuit |
| 13 | CCD |
| 14 | CCD driving circuit |
| 15 | function executing unit |
| 16 | RF transmitting unit |
| 17 | transmitting antenna unit |
| 18 | system controlling circuit |
| 19 | receiving antenna unit |
| 20 | separating circuit |
| 21 | power reproducing circuit |
| 22 | booster circuit |
| 23 | capacitor |
| 24 | travel state information detecting circuit |
| 25 | permanent magnet |
| 28 | receiving unit |
| 29 | image processing unit |
| 30 | storage unit |
| 31 | oscillator 31 |
| 32 | travel state information generator |
| 33 | multiplexing circuit |
| 34 | amplifier circuit |
| 35 | power supplying unit |
| 36 | reference detector selector |
| 37 | selector |
| 38 | distance calculator |
| 39 | position calculator |
| 40 | orientation direction database |
| 41 | orientation direction detector |
| 43 | test capsule |
| 44 | travel state processor |
| 45 | casing |
| 46 | permanent magnet |
| 47 | filling member |

48       storage unit

BEST MODE(S) FOR CARRYING OUT THE INVENTION

Exemplary embodiments of a system for detecting a travel state of a capsule endoscope in a subject relating to the present invention will be explained in detail below with reference to the accompanying drawings. It should be noted that the drawings are schematic ones and the relative relationship between the thickness and width of each of parts and the thickness ratio thereof, and the like are different from real ones. Obviously, the drawings include parts having different relationships of dimensions and ratios.

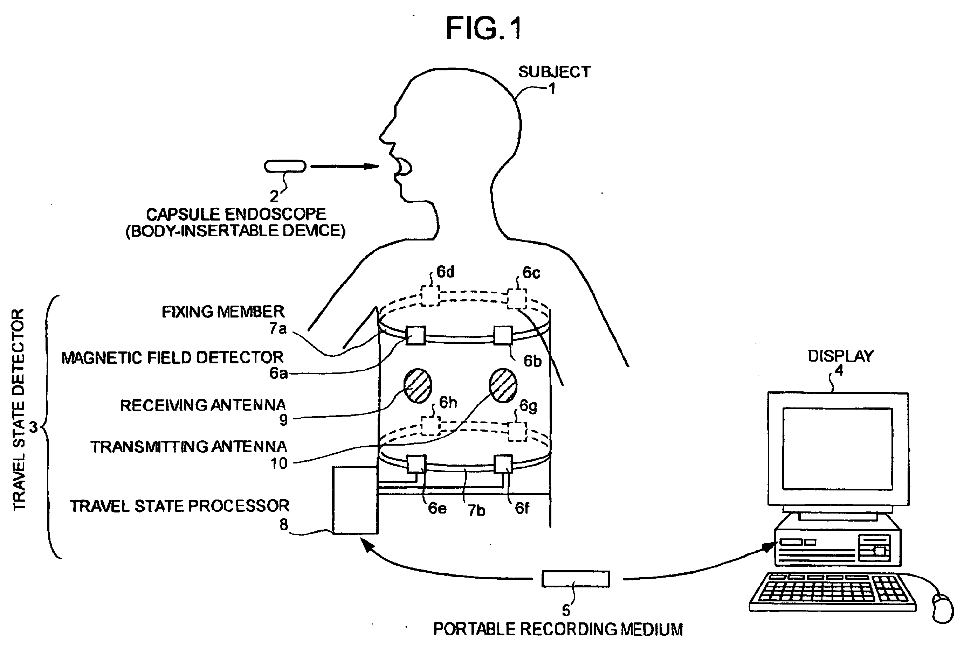

FIG. 1 is a schematic view of a system for detecting a travel state of a capsule endoscope in a body of a subject according to an embodiment. As shown in FIG. 1, the system for detecting a travel state of a capsule endoscope according to the embodiment of the present invention includes a capsule endoscope 2, once being swallowed, travels though a subject 1 and functions as an example of an body-insertable device in the body of the subject; a travel state detector 3 that detects, for example, the travel state of the capsule endoscope 2 in the body of the subject 1; a display 4 that views an image of an inside of the body of the subject 1 captured by the capsule endoscope 2; and a portable recording medium 5 for transferring information between the travel state detector 3 and the display 4.

The display 4 is used for displaying an image and the like of the body of the subject 1 captured by the capsule endoscope 2 and performs a similar function as that of a workstation and the like that displays an image based on data obtained from the portable recording medium 5. Specifically, a cathode-ray tube (CRT) display, a liquid crystal display, and the like may be used for the display 4 to directly view an image or to output an image to another medium, for example, a printer.

The portable recording medium 5 can be inserted/removed to/from a travel state processor 8 that will be explained later and the display 4, and has a structure that allows retrieving and recording of information when inserted to the travel state processor 8 and the display 4. Specifically, the portable recording medium 5 is inserted into the travel state processor 8 to record information on the position of the capsule endoscope 2 while the capsule endoscope 2 travels in the body cavity of the subject 1. After the capsule endoscope 2 is excreted from the body of the subject 1, the portable recording medium 5 is removed from the travel state processor 8 and inserted into the display 4, by which the recorded data is read. Differently from the case where the travel state processor 8 and the display 4 are connected to one another through a cable, by transferring data between the travel state processor 8 and the display 4 using the portable recording medium 5 such as Compact Flash (registered trademark) memory, the subject 1 can move freely even while the capsule endoscope 2 is traveling through the body of the subject 1.

The capsule endoscope 2 traveling through the body of the subject 1, once being swallowed, has a plurality of means for serving as; a function executing unit that executes a predetermined function on the inside of the subject 1; a receiving apparatus that receives a radio signal transmitted from the travel state detector 3; and a magnetic field generator that outputs a constant magnetic field used by the travel state detector to capture the travel state of the capsule endoscope. Hereafter, the configuration of the capsule endoscope 2 will be explained for each of components corresponding to the individual means mentioned above.

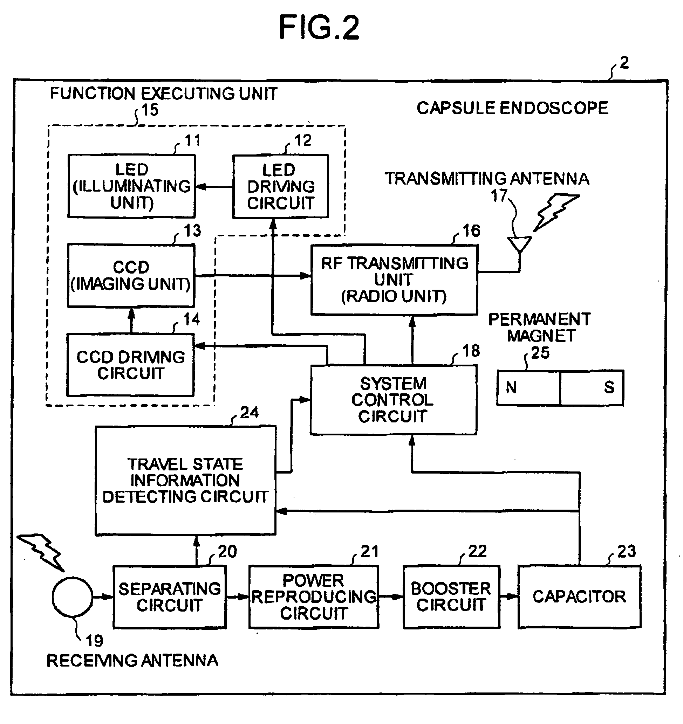

FIG. 2 is a schematic diagram of the structure of the capsule endoscope 2. The capsule endoscope 2 has a configuration so that the means serving as the function executing unit that executes the predetermined function; and a transmitter that transmits by radio information obtained by the function executing unit. Specifically, the capsule endoscope 2 includes a light-emitting diode (LED) 11, which is a illuminating means that illuminates an image pickup (imaging) region when capturing the image of the inside of the body of the subject 1, an LED driving circuit 12 which controls the drive state of the LED 11, a charge-coupled device (CCD) 13, which is an image capturing unit that captures a reflected light image from a region illuminated by the LED 11, and a CCD driving circuit 14 that controls the drive state of the CCD 13. The LED 11, the LED driving circuit 12, the CCD 13, and the CCD driving circuit 14 are defined as a whole to be a function executing unit 15 that performs given functions.

The capsule endoscope 2 includes an RF transmitting unit 16 that modulates image data captured by the CCD 13 and generates an RF signal, a transmitting antenna unit 17, which is a radio unit that transmits by radio an RF signal output from the RF transmitting unit 16, and a system controlling circuit 18 that controls operations in the LED driving circuit 12, the CCD driving circuit 14, and the RF transmitting unit 16.

By incorporating these mechanisms, the capsule endoscope 2 captures image data on an inspected region illuminated by the LED 11 by means of the CCD 13 while the capsule endoscope stays in the body of the subject 1. The captured image data is converted into an RF signal in the RF transmitting unit 16, and then transmitted to the outside of the body via the transmitting antenna unit 17.

The capsule endoscope 2 has a configuration to receive a radio signal transmitted from the travel state detector 3. Specifically, the capsule endoscope 2 includes a receiving antenna unit 19 that receives the radio signal transmitted from the travel state detector 3, and a separating circuit 20 that separates a power supplying signal from the signal received by the receiving antenna unit 19. The capsule endoscope 2 further includes a power reproducing circuit 21 that reproduces power from the separated power supplying signal, a booster circuit 22 that boosts the reproduced power, and a capacitor 2-3 that stores the boosted power. The capsule endoscope 2 further includes a travel state information detecting circuit 24 that detects contents of a travel state information signal from a component separated from the power supplying signal at the separating circuit 20, and outputs the detected travel state information signal to the system control circuit 18. Here, the travel state information is information on the travel state of the capsule endoscope 2 and derived by the travel state processor 8. The system control circuit 18 has a function of controlling the illumination interval of the LED 11 and the image pickup (imaging) interval of the CCD 13 based on the travel state information.

By incorporating these mechanisms, the capsule endoscope 2 first receives the radio signal transmitted from the travel state detector 3 at the receiving antenna unit 19, and separates the power supplying signal and the travel state information signal from the received radio signal using the separating circuit 20.

The travel state information signal separated by the separating circuit 20 is input to the system control circuit 18 via the travel state information detecting circuit 24. The system control circuit 18 controls the drive states of the LED 11, the CCD 13 and the RF transmitting unit 16 based on the travel state information. Specifically, if the system control circuit 18 acquires the travel state information, for example, indicating that the capsule endoscope 2 has stopped its travel in the subject 1, the system control circuit 18 controls the CCD 13 and the LED 11 to temporarily stop their driving and thereby prevents duplicated image data from being captured. On the other hand, the power supplying signal is reproduced as power by the power reproducing circuit 21. The reproduced power is boosted to a potential suitable for the capacitor 23 by the booster circuit 22, and then stored in the capacitor 23.

The capsule endoscope 2 further has a configuration to serve as the magnetic field generator. Specifically, the capsule endoscope 2 includes a permanent magnet 25 that outputs the constant magnetic field used to detect the current position and travel state of the capsule endoscope 2. The permanent magnet 25 functions as a magnetic field generator defined in claims. The permanent magnet 25, which has such a size that the permanent magnet can be accommodated in the capsule endoscope 2, provides a function of outputting a constant magnetic field, in which a fluctuation of magnetic field intensity over time is negligible. Instead of the permanent magnet 25, for example, a coil supplied with a constant current to generate a constant magnetic field may be used as the magnetic field generator. If the permanent magnet 25 is used, however, there is an advantage that driving power is unnecessary. Therefore, it is preferred to form the magnetic field generator with the permanent magnet 25.

The constant magnetic field generated by the permanent magnet 25 is represented by closed curve lines of magnetic force which start from an N pole, travel through an external region of the capsule endoscope 2, and return to an S pole. The intensity of the constant magnetic field represented by the lines of magnetic force can be considered to depend only on the distance from the capsule endoscope 2. In other words, the size of the permanent magnet 25 incorporated in the capsule endoscope 2 is minute enough to be negligible as compared with the distance between the capsule endoscope 2 and magnetic field detectors 6a to 6h. Therefore, a magnetic field intensity P at a point that is a distance r apart from the capsule endoscope 2 is represented by the following equation using a proportional factor $\alpha$.

$$P = \alpha/r^3 \qquad\qquad (1)$$

The system according to the present embodiment detects the position of the capsule endoscope 2 based on the relationship represented by Equation (1) as described later. The travel direction of the constant magnetic field output from the permanent magnet 25 has a position-dependence. As explained later, the system according to the present embodiment has a configuration also to detect a direction of a longitudinal axis of the capsule endoscope 2 (hereafter referred to as "orientation") as one aspect of the position information using the position-dependence of the travel direction of the constant magnetic field.

Now, the travel state detector 3 will be explained. The travel state detector 3 is provided to detect the travel state of the capsule endoscope 2 in the body of the subject 1 based on the constant magnetic field output from the capsule endoscope 2. Specifically, as shown in FIG. 1, the travel state detector 3 includes magnetic field detectors 6a to 6h that detect the constant magnetic field output from the capsule endoscope 2, a fixing member 7a that fixes the magnetic field detectors 6a to 6d to the body of the subject 1, a fixing member 7b that fixes the magnetic field detectors 6e to 6h to the body of the subject 1, the travel state processor 8 that calculates the position of the capsule endoscope 2 based on the magnetic field intensities detected by the magnetic field detectors 6a to 6h, a receiving antenna 9 that receives a radio signal transmitted from the capsule endoscope 2, and a transmitting antenna 10 that transmits a radio signal to the capsule endoscope 2. The magnetic field detectors 6a to 6h, the receiving antenna 9, and the transmitting antenna 10 are electrically connected to the travel state processor, and configured so that information may be received from and transmitted to the travel state processor 8.

The magnetic field detectors 6a to 6h detect the magnetic field intensity and magnetic field direction at their respective locations. Specifically, each of the magnetic field detectors 6a to 6h is formed using, for example, a Magneto Impedance (MI) sensor. The MI sensor uses a configuration, for example, an FeCoSiB amorphous wire as a magneto-sensitive medium to detect the magnetic field intensity under favor of the MI effect, in which the magnetic impedance of the magneto-sensitive medium is fluctuates largely by the external magnetic field when a high frequency current is supplied through the magneto-sensitive medium. A different type of magnetic field sensor may be used as each of the magnetic field detectors 6a to 6h. If the MI sensor is used, however, there is an advantage that the magnetic field can be detected at a particularly high sensitivity. In the present embodiment, the magnetic field detectors 6a to 6h are disposed at positions respectively, thereby forming vertexes of a cube.

The fixing members 7a and 7b fix the magnetic field detectors 6a to 6h to the body of the subject 1. Specifically, each of the fixing members 7a and 7b is formed in an annular form using, for example, an elastic member, and configured so that it may be fixed in close contact with the trunk of the subject 1. The magnetic field detectors 6a to 6d and the magnetic field detectors 6e to 6h are fixed to the trunk of the subject 1 in predetermined positions by the fixing members 7a and 7b, respectively. By fixing the fixing members 7a and 7b to the trunk of the subject 1 in close contact, the magnetic field detectors 6a to 6h may be disposed at fixed to the trunk of the subject 1 without loosing.

The receiving antenna 9 receives a radio signal transmitted from the capsule endoscope 2. As explained later, the capsule endoscope 2 has a function of picking up an image of the inside of the body of the subject 1 and transmitting the image to the outside of the body of the subject by radio. The receiving antenna 9 receives a radio signal transmitted from the capsule endoscope 2 and outputting it to the travel state processor 8. Specifically, the receiving antenna 9 includes, for example, a loop antenna and a fixing unit that fixes the loop antenna to the trunk of the subject 1.

The transmitting antenna 10 transmits a signal generated by the travel state processor 8 to the capsule endoscope 2. As explained later, the travel state processor 8 in the present embodiment superposes the power supplying signal that serves as drive power for the capsule endoscope 2 on the travel state information signal concerning the travel state of the capsule endoscope 2 and then outputs a resultant signal to the capsule endoscope 2. The transmitting antenna 10 transmits these signals to the capsule endoscope 2 by radio. Specifically, the transmitting antenna 10 includes, for example, a loop antenna and a fixing unit that fixes the loop antenna to the trunk of the subject 1.

Now, the travel state processor 8 will be explained. The travel state processor 8 has a function for receiving a radio signal transmitted from the capsule endoscope 2, a function for transmitting a predetermined signal to the capsule endoscope 2 by radio, and a function for calculating the position and the orientation direction of the capsule endoscope 2 and further determining the travel state of the capsule endoscope 2. Hereafter, a configuration of the travel state processor 8 will be explained for every component corresponding to each function.

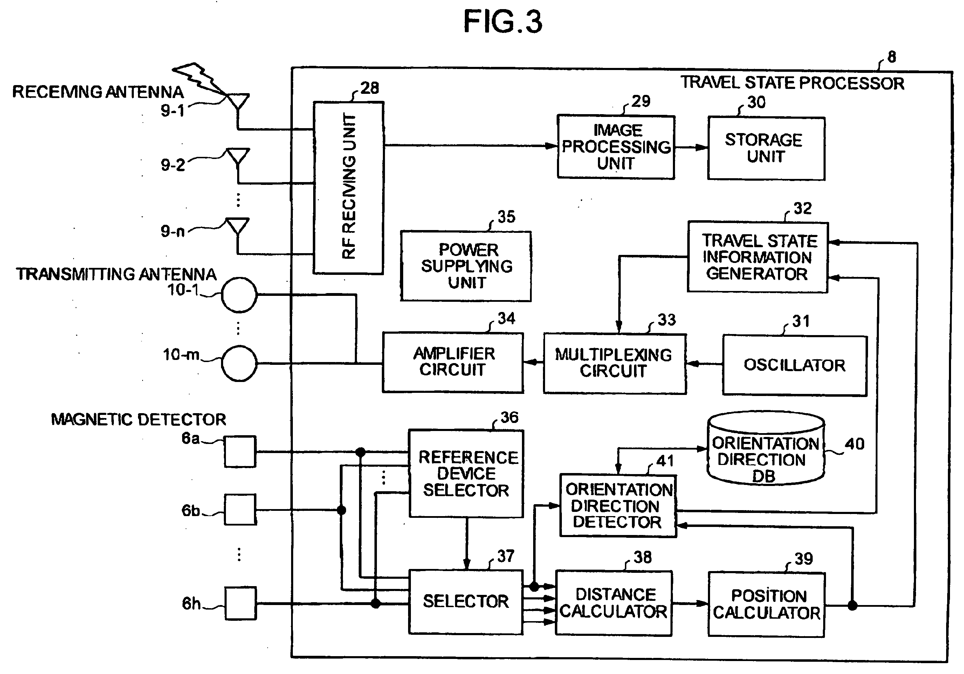

FIG. 3 is a block diagram of a whole configuration of the travel state processor 8. First, the travel state processor 8 is configured as a receiving apparatus that receives image data captured inside of body of the subject 1 transmitted from the capsule endoscope 2 by radio. Specifically, the travel state processor 8 includes an RF receiving unit 28 that conducts predetermined processing such as demodulation on a radio signal received by a selected receiving antenna and extracts image data acquired by the capsule endoscope 2 from the radio signal, an image processing unit 29 that conducts necessary processing on output image data, and a storage unit 30 that stores image data after being image-processed.

The travel state processor 8 is configured so that it may serve as a transmitter that generates the power supplying signal and the travel state information signal to be transmitted to the capsule endoscope 2 and outputs the signals to transmitting antennas 10-1 to 10-m. Specifically, as shown in FIG. 3, the travel state processor 8 includes an oscillator 31 having a function of generating the power supplying signal and a function of defining an oscillating frequency, a travel state information generator 32 that generates the travel state information signal explained later, a multiplexing circuit 33 that combines the power supplying signal and the travel state information signal, and an amplifier circuit 34 that amplifies the strength of the combined signal. The signal amplified by the amplifier circuit 34 is sent to the transmitting antennas 10-1 to 10-m, and transmitted to the capsule endoscope 2. The travel state processor 8 includes a power supplying unit 35 having any of given capacitor, an AC power adaptor, and the like. Components of the travel state processor 8 use power supplied from the power supplying unit 35 as driving energy.

The travel state processor 8 is configured so that it may serve as a position calculator that calculates the position of the capsule endoscope in the body of the subject 1, which is needed when generating the travel state information. Specifically, the travel state processor 8 includes a reference detector selector 36 that selects a reference magnetic field detector (hereafter referred to as "reference detector") from among the magnetic field detectors 6a to 6h, and a magnetic selector 37 that outputs a magnetic field intensity obtained in a predetermined number of magnetic field detectors based on a result of selection conducted by the reference detector selector 36. The travel state processor 8 further includes a distance calculator 38 that calculates the distance between the capsule endoscope 2 and the reference detector, and a position calculator 39 that calculates the position of the capsule endoscope 2 by conducting computation processing using the calculated distance and the position coordinates of the reference detector used to calculate the distance.

The reference detector selector 36 selects the magnetic field detector with the largest value for the detected magnetic field intensity from the magnetic field detectors 6a to 6h. Specifically, the reference detector selector 36 compares the magnetic field intensity values output from the magnetic field detectors 6a to 6h with each other, selects the magnetic field detector (reference detector) that outputs the largest magnetic field intensity value, and then information specifying the reference detector (for example, information indicating which is the reference detector among the magnetic field detectors 6a to 6h) to the magnetic selector 37.

The magnetic selector 37 selects a plurality of magnetic field detectors based on the result of selection of the reference detector selector 36 and outputs the magnetic field intensities obtained by the selected magnetic field detectors (selected detectors) and the magnetic field intensity obtained by the reference detector to the distance calculator 38. Specifically, the magnetic selector 37 selects three magnetic field detectors disposed in directions orthogonal to each other with respect to the reference detector. Namely, in the system for detecting the travel state according to this embodiment, as also shown in FIG. 1, the magnetic field detectors 6a to 6h are disposed so as to form vertexes of a cube, so that three magnetic field detectors positioned in direction orthogonal to each other always exist for any magnetic field detector, and the magnetic selector 37 selects the three magnetic field detectors as selected devices.

The distance calculator 38 calculates the distances from the reference detector, and the selected devices, to the capsule endoscope 2 based on the magnetic field intensities received via the magnetic selector 37. Specifically, the distance calculator 38 calculates the distance between the magnetic field detector that has detected the magnetic field intensity and the capsule endoscope 2 by performing the computing process shown by Equation (1) on the input magnetic field intensity.

The position calculator 39 calculates the position of the capsule endoscope 2 by performing a predetermined computing process based on the distance between the magnetic field detector selected as a reference detector and the capsule endoscope 2. The position calculator 39 also, after calculating the position of the capsule endoscope 2, outputs the result of calculation to the storage unit 30.

The travel state processor 8 is configured so that it may serve as an orientation direction detector that detects the orientation direction of the capsule endoscope 2, which is needed when generating the travel state information. Specifically, the travel state processor 8 includes an orientation direction database 40 that stores information concerning the orientation direction, and an orientation direction detector 41 that detects the orientation direction of the capsule endoscope 2 based on a magnetic field direction detected by a predetermined magnetic field detector 6. The orientation direction database 40 stores in advance data concerning the magnetic field intensity received by the magnetic field detector 6 and the orientation direction of the capsule endoscope 2 with respect to the relationship in position between the magnetic field detector 6 and the capsule endoscope 2. The principles of operation of the orientation direction database 40 and the orientation direction detector 41 are explained in detail later.

The travel state processor 8 is configured so that it may derive the travel state based on information on the calculated position of the capsule endoscope 2 and the orientation direction. Specifically, the travel state processor 8 includes the travel state information generator 32, and derives the travel state of the capsule endoscope 2 and generates the travel state information by using the travel state information generator 32.

Now, the principle of operation of the system for detecting the travel state according to the present embodiment is explained. Hereinafter, the operations of position calculation, orientation direction calculation, and travel state information generation concerning the capsule endoscope 2 conducted by the travel state processor 8 will be explained in sequence.

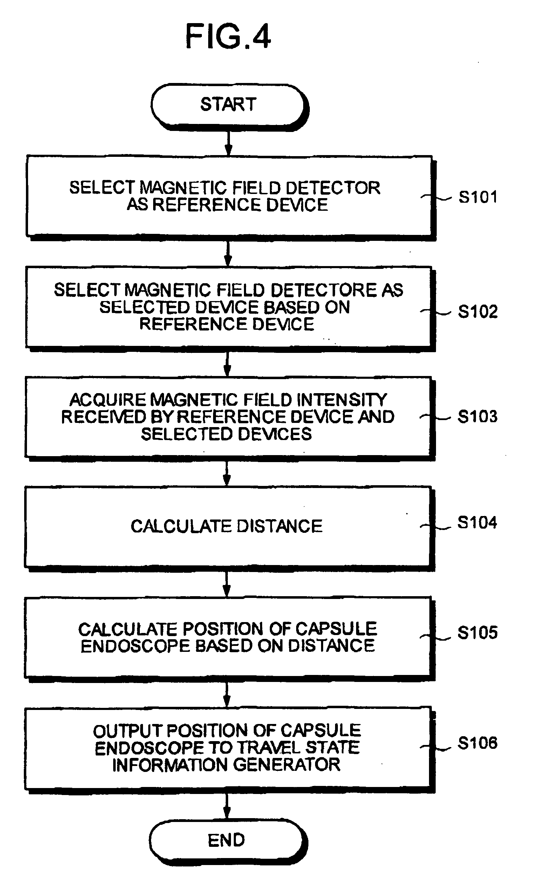

The operation of the travel state processor 8 to calculate the position of the capsule endoscope 2 will be explained. FIG. 4 is a flowchart of the position calculation operation of the travel state processor 8, and

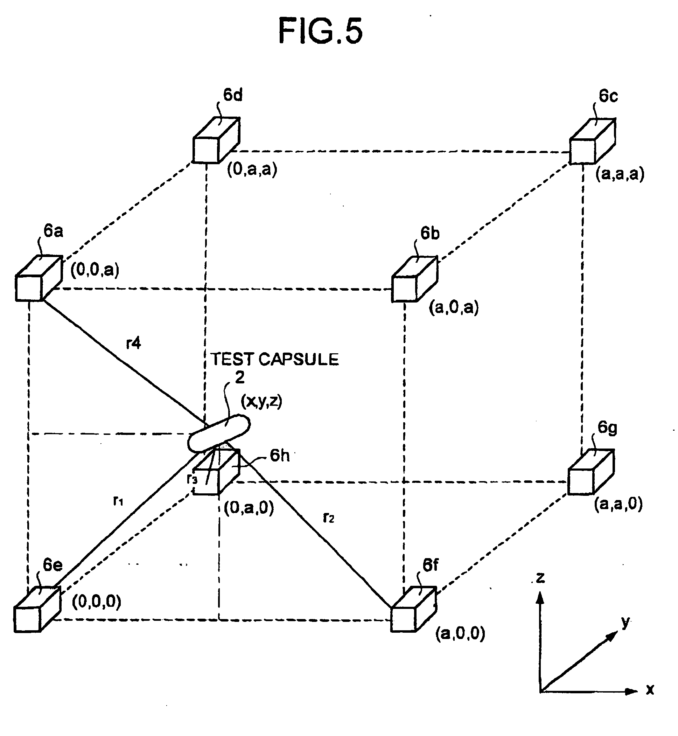

FIG. 5 is a schematic diagram for explaining the algorithm of the position calculation. In FIG. 5, the length of one side of a cube formed by the magnetic field detectors 6a to 6h is assumed to be "a". As is explained later, the position of the magnetic field detector 6e selected as the reference detector (detector) is assumed to be the origin, the direction from the magnetic field detector 6e toward the magnetic field detector 6f to be an x direction, the direction from the magnetic field detector 6e toward the magnetic field detector 6h to be a y direction, and the direction from the magnetic field detector 6e toward the magnetic field detector 6a to be a z direction, respectively. The positions of the magnetic field detectors 6a to 6h are determined based on the xyz coordinate system, and the position of the capsule endoscope 2 in the xyz coordinate system is expressed by means of (x,y,z). Referring to FIGS. 4 and 5, if appropriate, the operation of the travel state processor 8 is explained below.

First, the travel state processor 8, using the reference detector selector 36, selects the magnetic field detector with the highest one among the magnetic field intensities received by the magnetic field detectors 6a to 6h (step S101). In the example of FIG. 5, the magnetic field detector 6e is selected as the magnetic field detector detecting the highest magnetic field intensity. In the following description, it is also assumed that the magnetic field detector 6e is the reference detector.

The travel state processor 8 selects three devices using the magnetic selector 37 based on the reference detector selected in step S101 (step S102), and outputs the magnetic field intensities obtained by the reference detector and the selected detectors to the distance calculator 38 (step S103). In the example of FIG. 5. the magnetic field detectors 6f, 6h, and 6a are disposed in the directions orthogonal to each other with respect to the magnetic field detector 6e, the reference detector, so that the magnetic selector 37 may select the magnetic field detectors 6f, 6h, and 6a, selected detectors.

Next, the travel state processor 8 calculates the distance from the capsule endoscope 2 based on the magnetic field intensity obtained by the reference detector selected in step S101 and the magnetic field intensities obtained by the detectors selected in step S102 by the distance calculator 38 (step S104). Specifically, the distance calculator 38 calculates the distance by evaluating Equation (1) using the magnetic field intensity input via the magnetic selector 37. In the example of FIG. 5, the distance calculator 38 calculates distances $r_1$, $r_2$, $r_3$, and $r_4$ between the capsule endoscope 2 and the magnetic field detectors 6e, 6f, 6h, and 6a, respectively, based on the magnetic field intensities detected by the reference detector and the selected detectors.

The travel state processor 8 calculates the position of the capsule endoscope 2 by performing the computing process in the position calculator 39 (step S105). Specifically, since the position of the capsule endoscope 2 is calculated by finding the x, y, and z coordinates of the capsule endoscope 2, the coordinates of the capsule endoscope 2 are derived using the coordinates of the magnetic field detectors 6e, 6f, 6h, and 6a and the values of distances calculated in step S104.

For example, the position coordinates (x,y,z) of the capsule endoscope 2 can be geometrically derived from the relationship in position shown in FIG. 5 and, specifically, can be calculated by evaluating the following equations.

$$(x-0)^2 + (y-0)^2 + (z-0)^2 = r_1^2 \qquad (2)$$

$$(x-a)^2 + (y-0)^2 + (z-0)^2 = r_2^2 \qquad (3)$$

$$(x-0)^2 + (y-a)^2 + (z-0)^2 = r_3^2 \qquad (4)$$

$$(x-0)^2 + (y-0)^2 + (z-a)^2 = r_4^2 \qquad (5)$$

In Equations (2) to (5), three unknown letters are found so that three equations are theoretically sufficient. In detecting the actual position, however, to suppress deterioration in accuracy of the position detection of the capsule endoscope 2 due to positional deviations of the magnetic field detectors 6a to 6h, a distance derivation error, and the like, after solving Equations (2) to (5), the coordinates of the magnetic field detector, and the like are corrected so that the values x, y, and z may be evaluated into the fixed values.

Finally, the travel state processor 8 outputs the information on the position of the capsule endoscope 2 calculated by the position calculator 39 to the travel state information generator 32 (step S106). The output information on the position of the capsule endoscope 2 is used to derive the travel state of the capsule endoscope 2 explained later.

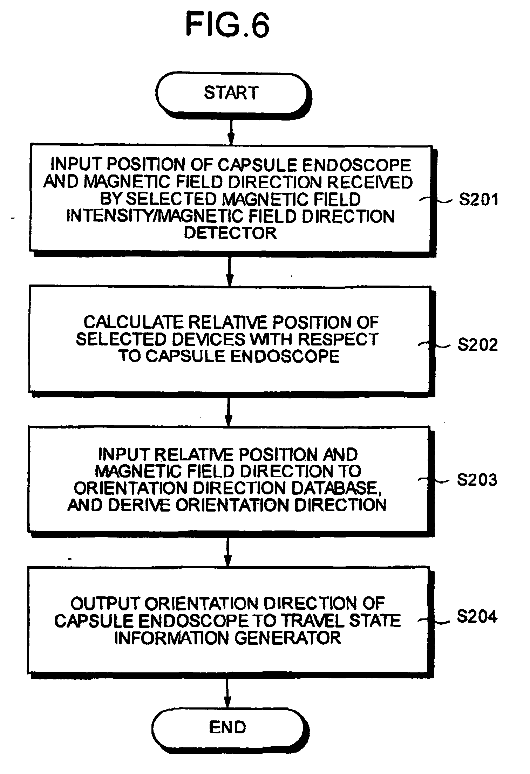

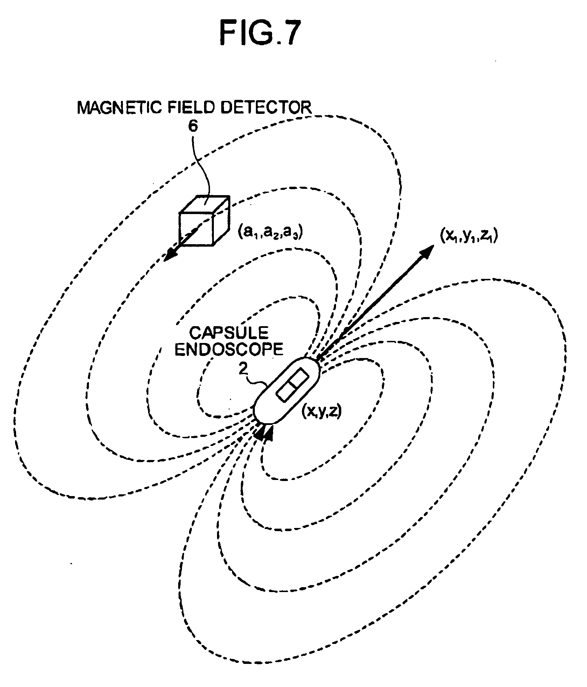

Now, the operation of deriving the orientation direction of the capsule endoscope 2 performed by the travel state detector 3 is explained. FIG. 6 is a flowchart of the principle of operation of deriving the orientation direction. FIG. 7 is a schematic diagram of the operation of deriving the orientation direction. Referring to FIGS. 6 and 7, if appropriate, the operation of deriving the orientation direction for the capsule endoscope 2 is explained.

First, the orientation direction detector 41 inputs the position of the capsule endoscope 2, and a magnetic field direction received by a magnetic field detector 6 selected from among the magnetic field detectors 6a to 6h (step S201). Any algorithm may be used to select the magnetic field detector 6. In the present embodiment, however, for example, a magnetic field detector 6 with the greatest magnetic field intensity received is selected. In an example shown in FIG. 7, the orientation direction detector 41 detects coordinates (a1, a2, a3) of the selected magnetic field detector 6 and a magnetic field direction represented by a directed vector indicated by an arrow.

The orientation direction detector 41 calculates a relative position of the magnetic field detector 6 selected at the step S201 with respect to the capsule endoscope 2 (step S202). Specifically, the orientation direction detector 41 is supplied with the position of the capsule endoscope 2 calculated by the position calculator 39 to derive relative coordinates of the magnetic field detector 6 selected at the step S201 with respect to the capsule endoscope 2. In the example shown in FIG. 7, relative position coordinates ($a_1$-x, $a_2$-y, $a_3$-z) of the magnetic field detector with the position of the capsule endoscope 2 assumed to be the origin are derived based on the coordinates ($a_1$, $a_2$, $a_3$) of the magnetic field detector 6 and the coordinates (x, y, z) of the capsule endoscope 2.

Thereafter, the orientation direction detector 41 inputs the magnetic field direction received at the step S201 and the relative position of the magnetic field detector 6 selected at the step S202 to the orientation direction database 40, and acquires data on the orientation direction of the capsule endoscope 2 (step S203). As shown in FIG. 7, the direction of the constant magnetic field output from the permanent magnet 25 contained in the capsule endoscope 2 has a property that it uniquely depends on the orientation direction of the capsule endoscope 2 and the relative position with respect to the capsule endoscope 2. Therefore, the orientation direction of the capsule endoscope 2, the relative coordinates with respect to the capsule endoscope 2, and the direction of the constant magnetic field at the relative coordinates are stored in the orientation direction database 40 so as to be associated with each other. For this reason, the orientation direction of the capsule endoscope 2 can be extracted by inputting the relative coordinates of the magnetic field detector 6 and the detected direction of the constant magnetic field to the orientation direction database 40. In the example shown in FIG. 7, it is derived that the orientation direction of the capsule endoscope 2 is $(x_1, y_1, z_1)$ based on the output result of the orientation direction database 40.

The orientation direction detector 41 outputs acquired information on the orientation direction of the capsule endoscope 2 to the travel state information generator 32 (step S204). The output information on the orientation direction of the capsule endoscope 2 is used to derive the next travel state of the capsule endoscope 2.

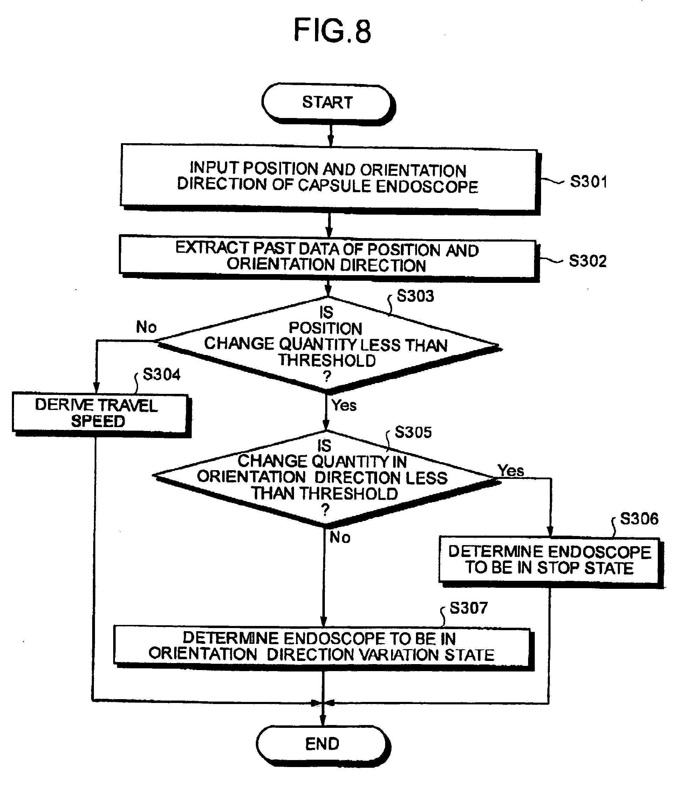

Next, the operation of deriving the travel state of the capsule endoscope 2 based on the derived position and the orientation direction is explained. FIG. 8 is a flowchart of the operation of deriving the travel state performed by the travel state information generator 32. Referring to FIG. 8, is appropriate, the operation of deriving the travel state is explained below.

First, the travel state information generator 32 inputs information on the position of the capsule endoscope 2 calculated by the position calculator 39 and information on the orientation direction of the capsule endoscope 2 derived by the orientation direction detector 41 (step S301). The travel state information generator 32 extracts already stored data on the previous position and the previous orientation direction of the capsule endoscope 2, to compare these data with the received information on the position and the orientation direction (step S302).

Thereafter, the travel state information generator 32 calculates a variation in position (= travel distance) of the capsule endoscope 2, and determines whether the calculated variation in position is less than a predetermined threshold (step S303). If the variation in position is determined to be lower than the threshold, the procedure proceeds to step 5305. If the variation in position is determined to be higher than or equal to the threshold, the procedure proceeds to step S304.

If the variation in position is determined to be higher than or equal to the threshold, the travel speed of the capsule endoscope 2 is further calculated (step S304). The system for detecting the travel state according to the present embodiment is configured so that the function executing unit 15 in the capsule endoscope 2 may capture the images. The image pickup interval, therefore, is calculated with reference the travel speed of the capsule endoscope 2. Once the travel speed has been calculated, the result is established as the travel state information, output to the multiplexing circuit 33, and transmitted to the capsule endoscope 2 as a radio signal. At this point, the whole procedure has finished.

On the other hand, if the variation in position is determined to be lower than a threshold, the travel state information generator 32 calculates a variation in the orientation direction of the capsule endoscope 2, and determines whether the calculated variation in position is lower than a threshold (step S305). If the variation in position in the orientation direction is determined to be lower than the threshold, the travel state information generator 32 determines the capsule endoscope 2 to be in the stop state (step S306), generates travel state information indicating it, and outputs the generated travel state information to the multiplexing circuit 33.

If the variation in position in the orientation direction is determined to be higher than or equal to the threshold at the step S305, the travel state information generator 32 determines that the capsule endoscope 2 is in an orientation direction variation state (step 5307), generates travel state information indicating it, and outputs the generated travel state information to the multiplexing circuit 33. As heretofore explained, the travel state information generator 32 determines the travel speed of the capsule endoscope 2, whether the capsule endoscope 2 is not traveling but its orientation direction is varying, or whether the capsule endoscope 2 has completely stopped its travel, based on the result of the position detection and the orientation direction detection. The travel state information generator 32 outputs a result of the determination as travel state information.

Now, advantages of the system for detecting the travel state according to the present embodiment will be property in which the intensity attenuates nearly uniquely regardless of variations in physical parameters such as the dielectric constant and permeability in the propagation region. Therefore, the system according to the present embodiment has a feature that the relationship represented by Equation (1) holds satisfactorily. Even in position detection in a space where for example, objects such as internal organs of the human body that differ from each other in physical parameters are present, the system according to the present embodiment has an advantage in that the position can be detected with higher accuracy as compared with the position detection using electromagnetic waves or the like.

The system according to the present embodiment is configured so that the orientation direction of the capsule endoscope 2 may be detected based on the constant magnetic field output from the permanent magnet 25. In the same way as the position detection, the constant magnetic field output from the permanent magnet 25 is not only hardly susceptible to the influence of organs and the like in the subject 1, but also has a characteristic that the magnetic field direction in a predetermined position is determined largely depending on the orientation direction of the capsule endoscope 2 and the relative position with respect to the capsule endoscope 2. The direction of the lines of magnetic force output from the permanent magnet 25 has a characteristic that it is nearly uniquely determined regardless of the presence of objects such as internal organs that differ from each other in physical parameters. Even while the capsule endoscope 2 is traveling in the subject 1, therefore, the orientation direction can be derived accurately by deriving the orientation direction based on the travel direction of the constant magnetic field output from the permanent magnet 25.

The system for detecting the travel state according to the present embodiment derives the travel state of the capsule endoscope 2 based on the accurately calculated position and orientation direction of the capsule endoscope 2. In cooperating this function, there is an advantage that, for example, the doctor or the like can recognize how the capsule endoscope 2 travels through the subject 1.

In the present embodiment, the drive state of the function executing unit 15 is controlled based on the derived travel state of the capsule endoscope 2. In the present embodiment, the function executing unit 15 has the illumination means and the image pickup means. Images in the body of the subject 1 can be acquired efficiently by controlling the drive state of the function executing unit 15 based on the travel state. For example, if travel state information indicating that the capsule endoscope 2 has stopped traveling in the subject 1 is obtained, it is possible to prevent duplicated image data of the same region from being acquired, by stopping the drive of the LED 11 and the CCD 13. When the capsule endoscope 2 travels faster than the ordinary speed as in passing through the esophagus in the subject 1, it becomes possible to acquire sufficient image data by narrowing the intervals between the image pickup operations.

In the present embodiment, the change in the orientation direction of the capsule endoscope 2 is also derived as the travel state information. For example, when the orientation direction of the capsule endoscope 2 is changing although it stays in the same region, therefore, the situation can be detected. In such a travel state, the field of vision in image pickup of the CCD 13 varies although there is no variation in position for the capsule endoscope 2, and consequently more information in the body of the subject 1 can be acquired by conducting the image pickup operation. The system according to the present embodiment can be configured so that it may perform image pickup operation, for example, when it has determined that the capsule endoscope 2 stops in the subject 1 and the variation in position in orientation direction is higher than or equal to a predetermined threshold. As a result, the system according to the present embodiment has an advantage in that more information can be acquired on the images in the body of the subject 1.

In the system for detecting the travel state according to the present embodiment, the magnetic field detectors 6a to 6h are used in detecting the position and orientation direction of the capsule endoscope 2. Therefore, there is an advantage in that the system can be implemented in a simple configuration. In other words, the system according to the present embodiment detects the position and the orientation direction of the capsule endoscope 2 using the common detector but not individual different means. Therefore, the detecting mechanism may be configured in a simple manner, resulting in an advantage of a reduced manufacture cost.

A variant of the system according to the present embodiment will be explained. The system according to the present variant relates to a test capsule used when conducting preliminary examination of the inside of the subject to observe the presence of an isthmus or the like that would hamper a smooth travel of the capsule endoscope. In other words, the system according to the present variant is intended to observe how the test capsule travels through the body of the subject.

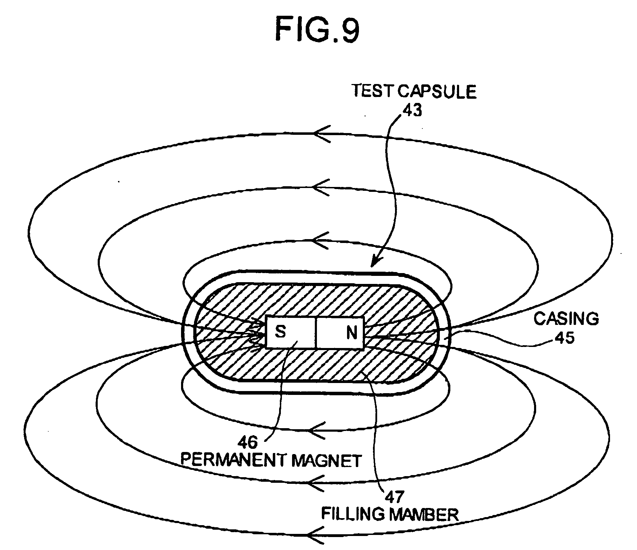

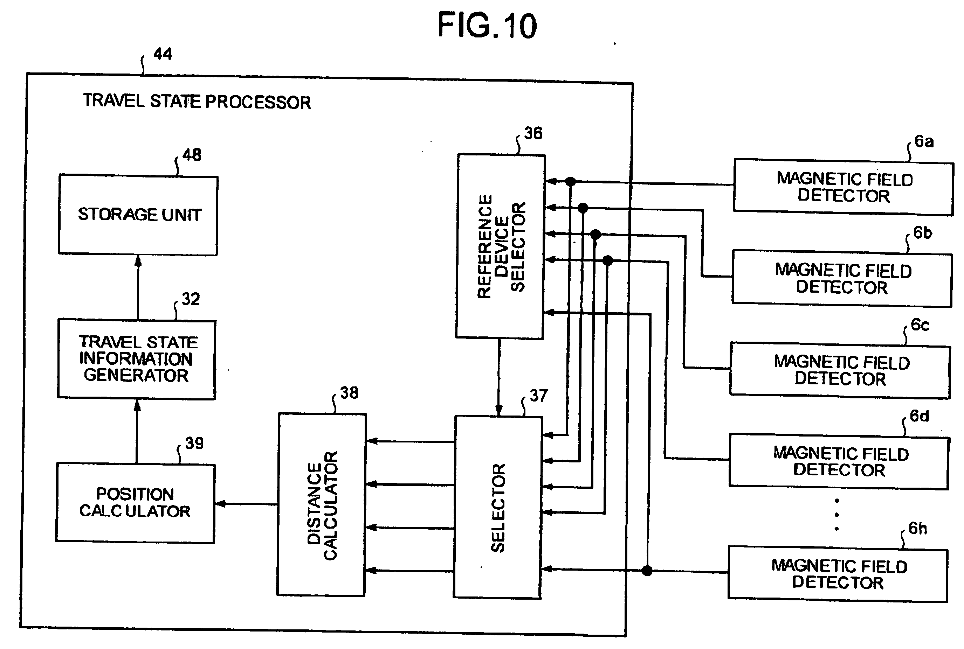

FIG. 9 is a schematic diagram of a whole configuration of a test capsule 43 as a component of the system according to the present variant. FIG. 10 is a schematic diagram of a whole configuration of a travel state detector contained in the system according to the present variant.

As shown in FIG. 9, the test capsule 43 includes a casing 45 having a capsule shape similar to that of the casing of the capsule endoscope, a permanent magnet 46 disposed inside the casing 45, and a filling member 47 used to fill a clearance between the casing 45 and the permanent magnet 46.

The casing 45 is formed of a soft deformable material. The casing 45 has a property of being degraded when it stays in the subject 1 for a certain period of time. The possibility of the casing 45 being degraded in the subject 1 brings about an advantage that it is not necessary to conduct an abdominal operation on the subject 1 even if the test capsule 43 introduced into the subject 1 should not be excreted to the outside of the body of the subject 1.

The filling member 47 fills the clearance between the casing 45 and the permanent magnet 46. The material used for the filling member 47 needs to be a material that does not exert a negative influence upon the subject 1. It is desirable to use, for example, a physiological saline solution or barium sulphate as the material. In particular, when the filling member 47 is formed of barium sulphate, there is an advantage in that the position of the test capsule 43 can be detected by X-ray inspection using the filling member 47 as a contrast medium.

A travel state processor 44 will be explained. As shown in FIG. 10, the travel state processor 44 includes a reference detector selector 36 that selects a reference detector from among the magnetic field detectors 6a to 6h, a magnetic selector 37 that selects selected devices based on the selected reference detector and outputs a magnetic field intensities obtained by the reference detector and the selected devices, a distance calculator 38 that calculates the distance from the test capsule 43 based on the magnetic field intensities output from the magnetic selector 37, a position calculator 39 that calculates the position of the test capsule 43 based on the calculated distance, and a travel state information generator 32 that generates travel state information based on the calculated position. The travel state processor 44 further includes a storage unit 48 that stores the travel state information generated by the travel state information generator 32. By outputting travel state information to the display 4 via the storage unit 48 and the portable recording medium 5, the doctor or the like may be recognize the travel state of the test capsule 43. In the present variant, the derived travel state information is limited only to the variation in position. As a matter of course, however, the variation in the orientation direction may also be derived in the same way as the embodiment.

Heretofore, the present invention is explained with reference to the embodiments and the variant. However, the present invention is not limited to the embodiments and the variant. Those skilled in the art may know various embodiments, variants and applications. For example, the system according to the present embodiment may be configured so that it may derive the orientation direction of the capsule endoscope 2 in the same way as that of the embodiment.

In the embodiments and the variant, a plurality of magnetic field detectors 6 are disposed on the body surface of the subject 1 so as to form vertexes of a cube, respectively. However, it is not necessary to limit the configuration to such an arrangement. In other words, as for the magnetic field detectors 6 and the like, it is only required that the relative positions with respect to the subject 1 have been previously recognized. Even if the magnetic field detectors 6 are not disposed in a cubic form, the position and the orientation direction can be detected based on the relative positions. Similarly, the number of the magnetic field detectors 6 is not necessary to restrict to eight. A system using a single magnetic field detector 6 can be configured as the simplest one. The capsule endoscope 2, as a device to be introduced into the subject, does not travel freely through the body of the subject 1, but travels on a route predetermined to some degree depending on the arrangement of internal organs such as the esophagus, stomach, the small intestine, and the colon. Therefore, it is possible to previously recognize the travel route of the device. The position of the device may be detected using the previously recognized route information and the intensity of the constant magnetic field received by the single magnetic field detector.

In the embodiments and variations, the reference detector and the selected devices are selected using the reference detector selector 36 and the magnetic selector 37, and the position is detected based on the magnetic field intensities detected by them. However, such a configuration is not indispensable for the present invention. For example, it is possible to calculate the distance from the capsule endoscope 2 to each of the magnetic field detectors 6a to 6h based on the detected intensities, establish eight equations similar to Equations (2) to (5), and calculate the position of the capsule endoscope 2 by evaluating these equations. In such a configuration, for example, computation using the least square method is possible and this results in an advantage that the derivation error in position of the capsule endoscope 2 can be further reduced.

Similarly, for example, in the embodiment, the orientation direction of the capsule endoscope 2 may be derived using a plurality of magnetic field detectors 6. In other words, it is also desirable to adopt a method, in which the orientation direction is derived in the manner as described above at a plurality of magnetic field detectors 6, to find an average of the obtained orientation directions, for example, thereby allowing for a more accurate derivation of orientation direction. The same holds for the position detection of the device to be introduced into the subject as well. It is possible to adopt a configuration in which position detection is conducted a plurality of times by using different combinations of the magnetic field detectors and respectively obtained positions are averaged.

Further, in the embodiment, the function executing unit 15 including the CCD 13, the image capturing unit, and the LED 11, the illuminating unit, has been explained. However, the function executing unit can be configured so that information on pH and the temperature in the subject 1 may be acquired. The device to be introduced into the subject may have an oscillator to acquire an ultrasonic wave image in the body of the subject 1. Still further, the device can be configured so that it may acquire a plurality of pieces of information from the intra-subject information.

The radio signal transmitted from the transmitting antennas 10-1 to 10-m need not be the travel state information signal superposed on the power supplying signal, and only the travel state information signal may be output. The travel state information signal may be superposed on a signal other than the power supplying signal. The travel state detector 3 can be configured so that it may receive only the radio signal output from the capsule endoscope 2. It is also possible to provide a storage unit in the capsule endoscope 2, and retrieve information from the storage unit after the capsule endoscope is excreted to the outside of the body of the subject 1.

INDUSTRIAL APPLICABILITY

As is clear from the foregoing, the system for detecting a travel state in a subject according to the present invention is useful in connection with the swallowable capsule endoscope employed for the medical treatment, and particularly suitable for a body-insertable device such as the capsule endoscope that performs position detection in a subject, such as a patient.

**Claims**

1. A system for detecting a travel state in a body of a subject (1), comprising:

a body-insertabte device (2) that is adapted to be swallowed, and after being swallowed to travel through a body of the subject (1); and
a travel state detector (3) for detecting the travel state of the body-insertable device (2) in the body of the subject (1), wherein
the body-insertable device (2) includes:

a constant magnetic field generator (25) for outputting a constant magnetic field to the outside of the body of the subject (1);
a function executing unit (15) that includes

an illuminating unit (11) for illuminating an inside of the body of the subject (1); and
an image pickup unit (13) for capturing an image of a region illuminated by the illuminating unit (11), obtaining information an inside of the subject (1); and a radio transmitting unit (16) that is adapted to transmit the information on the inside of the body of the subject (1) by radio,

the travel state detector (3) includes at least

a plurality of magnetic field detectors 6a-6h that are each disposed in a fixed position relative to the subject (1) and each of them is adapted to detect an intensity of a constant magnetic field output from the constant magnetic field generator (25);
a reference detector selector (36) that is adapted to select a reference magnetic field detector (6a-6h) from the plurality of magnetic field detectors (6a-6h), the reference magnetic field detector (6a-6h) being the magnetic field detector with the largest value for the detected intensity of magnetic field from the plurality of magnetic field detectors (6a-6h),
a magnetic selector (37) that is adapted to select a predetermined number of magnetic field detectors (6a-6h) from the plurality of magnetic field detectors (6a-6h) based on the result of selection of the reference detector selector (36)
a distance calculator (38) that is adapted to calculate a distance between the device and each of the selected magnetic field detectors (6a-6h) and the reference magnetic field detector based on the intensities of magnetic field detected by the magnetic field detectors (6a-6h) outputted by the magnetic selector (37), wherein the magnetic selector is adapted to butput the intensities of magnetic field obtained by the selected magnetic field detectors (6a-6h) and the intensity of magnetic field obtained by the reference magnetic field detector (6a-6h) to the disctance calculator
a position calculator (39) that is adapted to cacluate a position of the device (2) in the body of the subject (1) based on the calculated distances;
a travel state processor (32) that is adapted to determine a travel state of the device in the body of the subject (1) based on the position calculated by the position calculator (39), and
a receiving unit (9) that is adapted to receive a radio signal transmitted from the radio transmitting unit (16),

wherein the constant magnetic field generator (25) is disposed in the body-insertable device (2) such that a direction of the constant magnetic field is fixed, and

each of the magnetic field detectors (6a-6h) is adapted to detect a direction of the constant magnetic field generated from the constant magnetic field generator (25), and
the travel state detector (3) further includes an orientation direction detector (41) that is adapted to detect an orientation direction of the device in the body of the subject (1) based on the direction detected by each of the magnetic field detectors (6a-6h), and
the travel state processor (32) is adapted to determine whether a variation in position of the body-insertable device (2) is less than a first predetermined threshold, based on the position calculated by the position calculator (39), and to determine whether a variation in orientation direction of the body-insertable device (2) is less than a second predetermined threshold, based on the orientation direction detected by the orientation direction detector (41), and
if the variation in position is less than the first predetermined threshold, and the variation in orientation direction is less than the second predetermined threshold, then the travel state processor (32) is adapted to determine the body-insertable device (2) to be in a stopped state, and to transmit information indicating that the body-insertable device (2) is in the stopped state, to the body-insertable device (2) to cause the illuminating unit (11) and the image pickup unit (13) in the body-insertable device (2) to stop operation.

2. The system according to claim 1, wherein the travel state detector (3) further includes
an orientation direction database (40) that stores, in advance, a distance from the constant magnetic field generator (25), a relationship between a direction of the constant magnetic field, and an orientation direction of the device in the body of the subject (1), and
the orientation direction detector (41) is adapted to detect the orientation direction of the device in the body of the subject (1) using the orientation direction database (40).

3. The system according to claim 1, wherein the travel state detector (3) is adapted to generat travel state information to control a drive state of the function executing unit (15) contained in the body-insertable device (2) based on the travel state of the body-insertable device (2) in the body of the subject determined by the travel state processor (32).

**Patentansprüche**

1. System zum Ermitteln eines Bewegungszustandes in einem Körper einer Person (1), welches umfasst:

eine in einen Körper einbringbare Vorrichtung (2), die dazu geeignet ist, geschluckt zu werden und nachdem sie geschluckt wurde, sich durch einen Körper einer Person (1) fortzubewegen,
ein Bewegungszustand-Detektor (3) zum Ermitteln des Bewegungszustandes der in den Körper einbringbaren Vorrichtung (3) in dem Körper der Person, wobei die in den Körper einbringbare Vorrichtung (2) umfasst:

einen Konstantmagnetfeld-Generator (25) zum Ausgeben eines konstanten magnetischen Feldes außerhalb des Körpers der Person (1);
eine Funktionsausübungseinheit (15), die beinhaltet

eine Beleuchtungseinheit (11) zum Beleuchten des Körperinneren der Person (1); und

eine Bildaufnahmeeinheit (13) zum Aufnehmen eines Bildes eines von der Beleuchtungseinheit (11) beleuchteten Bereichs, zum Erhalten von Information des Körperinneren der Person (1); und eine Funkübertragungseinheit (16), die dazu geeignet ist, die Information über das Körperinnere der Person (1) per Funk zu übertragen,

wobei der Bewegungszustand-Detektor (3) wenigstens beinhaltet

eine Vielzahl von Magnetfelddetektoren (6a-6h), die jeweils in einer festen Lage bezüglich der Person (1) angeordnet sind, und die jeweils dazu geeignet sind, eine Intensität eines konstanten magnetischen Feldes, das von dem Konstantmagnetfeld-Generator (25) ausgegeben wurde, zu ermitteln;
ein Referenzdetektor-Selektor (36), der dazu geeignet ist, einen Referenzmagnetfelddetektor (6a-6h) aus der Vielzahl von Magnetfelddetektoren (6a-6h) auszuwählen, wobei der

Referenzmagnetfelddetektor (6a-6h) der Magnetfelddetektor mit dem größten Wert für die ermittelte Intensität des Magnetfelds aus der Vielzahl von Magnetfelddetektoren (6a-6h) ist,

ein magnetischer Selektor (37), der dazu geeignet ist, eine vorbestimmte Anzahl von Magnetfelddetektoren (6a-6h) aus der Vielzahl von Magnetfelddetektoren (6a-6h) basierend auf dem Ergebnis der Auswahl des Referenzdetektor-Selektors (36) auszuwählen,
ein Entfernungskalkulator (38), der dazu geeignet ist, eine Entfernung zwischen der Vorrichtung und jedem der ausgewählten Magnetfelddetektoren (6a-6h) und dem Referenzmagnetfelddetektor basierend auf den Magnetfeldintensitäten, die durch die Magnetfelddetektoren (6a-6h) ermittelt wurden und von dem magnetischen Selektor (37) ausgegeben wurden, zu berechnen, wobei der magnetische Selektor (37) dazu geeignet ist, die Magnetfeldintensitäten, die durch die ausgewählten Magnetfelddetektoren (6a-6h) erhalten wurden und die Magnetfeldintensität, die durch den Referenzmagnetfelddetektor erhalten wurde, an den Entfernungskalkulator (38) auszugeben,
ein Positionskalkulator (39), der dazu geeignet ist, eine Position der Vorrichtung (2) in dem Körper der Person (1) basierend auf den berechneten Entfernungen zu berechnen;

ein Bewegungszustand-Prozessor (32), der dazu geeignet ist, einen Bewegungszustand der Vorrichtung in dem Körper der Person (1) basierend auf der von dem Positionskalkulator (39) berechneten Position zu bestimmen, und

eine Empfangseinheit (9), die dazu geeignet ist, ein von der Funkübertragungseinheit (16) übertragenes Funksignal zu empfangen,

wobei der Konstantmagnetfeld-Generator (25) in der in den Körper einbringbaren Vorrichtung (2) angeordnet ist, so dass eine Richtung des konstanten magnetischen Feldes fest ist, und

jeder der Magnetfelddetektoren (6a-6h) eine Richtung des von dem Konstantmagnetfeld-Generator (25) erzeugten konstanten magnetischen Feldes ermittelt, und

der Bewegungszustand-Detektor (3) ferner einen

Orientierungsrichtungsdetektor (41) umfasst, der dazu geeignet ist, eine Orientierungsrichtung der Vorrichtung in dem Körper der Person (1) basierend auf der Entfernung, die von jedem der Magnetfelddetektoren (6a-6h) ermittelt wurde, zu ermitteln, und

der Bewegungszustand-Prozessor (32) geeignet ist zu bestimmen, ob eine Veränderung der Position der in den Körper einbringbaren Vorrichtung (2) weniger als ein erster vorbestimmter Schwellwert ist, basierend auf der vom Positionskalkulator (39) berechneten Position, und zu bestimmen, ob eine Veränderung der Orientierungsrichtung der in den Körper einbringbaren Vorrichtung (2) weniger als ein zweiter vorbestimmter Schwellwert ist, basierend auf der von dem Orientierungsrichtungsdetektor (41) ermittelten Orientierungsrichtung, und wenn die Veränderung der Position weniger als der erste vorbestimmte Schwellwert ist, und die Veränderung der Orientierungsrichtung weniger als der zweite vorbestimmte Schwellwert ist, der Bewegungszustand-Prozessor (32) dann geeignet ist zu bestimmen, dass die in den Körper einbringbare Vorrichtung (2) sich in einem angehaltenen Zustand befindet, und Information an die in den Körper einbringbare Vorrichtung (2) zu übertragen, die angibt, dass die in den Körper einbringbare Vorrichtung (2) sich in dem angehaltenen Zustand befindet, um die Beleuchtungseinheit (11) und die Bildaufnahmeeinheit (13) in der in den Körper einbringbaren Vorrichtung (2) zu veranlassen, den Betrieb einzustellen.

2. System nach Anspruch 1, wobei der Bewegungszustand-Detektor (3) ferner umfasst

eine Orientierungsrichtungdatenbank (40), die im Vorhinein eine Entfernung von dem Konstantmagnetfeld-Generator (25), eine Beziehung zwischen einer Richtung des konstanten magnetischen Feldes, und einer Orientierungsrichtung der Vorrichtung in dem Körper der Person (1) speichert, und

ein Orientierungsrichtungsdetektor (41), der dazu geeignet ist, die Orientierungsrichtung der Vorrichtung in dem Körper der Person (1) durch Benutzen der Orientierungsrichtungsdatenbank (40) zu ermitteln.

3. System nach Anspruch 1, wobei der Bewegungszustand-Detektor (3) dazu geeignet ist, Bewegungszustandsinformation zu erzeugen, um den Steuerungszustand der Funktionsausübungseinheit (15), die in der in den Körper einbringbaren Vorrichtung (2) enthalten ist, zu steuern, basierend auf dem von dem Bewegungszustand-Prozessor (32) ermittelten Bewegungszustand der in den Körper einbringbaren Vorrichtung (2) in dem Körper der Person.


**Revendications**

1. Système destiné à détecter un état de mouvement dans un corps d'un sujet (1) comprenant :

un dispositif pouvant être inséré dans le corps (2) qui est adapté pour être avalé, et après avoir été avalé, pour se déplacer à travers un corps du sujet (1) ; et

un détecteur d'état de déplacement (3) pour détecter l'état de déplacement du dispositif pouvant être inséré dans le corps (2) dans le corps du sujet (1), dans lequel

le dispositif pouvant être inséré dans le corps (2) comprend :

un générateur de champ magnétique constant (25) pour émettre un champ magnétique constant vers l'extérieur du corps du sujet (1) ;

une unité d'exécution de fonction (15) qui comprend

une unité d'éclairage (11) pour éclairer un intérieur du corps du sujet (1) ; et

une unité de collecte d'image (13) pour capturer une image d'une région éclairée par l'unité d'éclairage (11), obtenant des informations d'un intérieur du sujet (1) ; et une unité de transmission radio (16) qui est adaptée pour transmettre les informations sur l'intérieur du corps du sujet (1) par radio,

le détecteur d'état de déplacement (3) comprend au moins

une pluralité de détecteurs de champ magnétique (6a-6h) qui sont chacun disposés dans une position fixe par rapport au sujet (1) et chacun d'eux est adapté pour détecter une intensité d'une sortie de champ magnétique constante du générateur de champ magnétique constant (25) ;

un sélecteur de détecteur de référence (36) qui est adapté pour sélectionner un détecteur de champ magnétique de référence (6a-6h) de la pluralité de détecteurs de champ magnétique (6a-6h), le détecteur de champ magnétique de référence (6a-6h) étant le détecteur de champ magnétique avec la valeur la plus grande pour l'intensité de champ magnétique détectée de la pluralité de détecteurs de champ magnétique (6a-6h),

un sélecteur magnétique (37) qui est adapté pour sélectionner un nombre prédéterminé de détecteurs de champ magnétique (6a-6h) de la pluralité de détecteurs de champ magnétique (6a-6h) en se basant sur le résultat de sélection du sélecteur de détecteur de référence (36),

un calculateur de distance (38) qui est adapté pour calculer une distance entre le dispositif et chacun des détecteurs de champ magnétique (6a-6h) sélectionnés et le détecteur de champ magnétique de référence en se basant sur les intensités de champ magnétique détectées par les détecteurs de champ magnétique (6a-6h) et émises par le sélecteur magnétique (37), dans lequel le sélecteur magnétique est adapté pour émettre les intensités de champ magnétique obtenues par les détecteurs de champ magnétique (6a-6h) sélectionnés et l'intensité de champ magnétique obtenue par le détecteur de champ magnétique de référence (6a-6h) par rapport au calculateur de distance,

un calculateur de position (39) qui est adapté pour calculer une position du dispositif (2) dans le corps du sujet (1) en se basant sur les distances calculées ;

un processeur d'état de déplacement (32) qui est adapté pour déterminer un état de déplacement du dispositif dans le corps du sujet (1) en se basant sur la position calculée par le calculateur de position (39), et une unité de réception (9) qui est adaptée pour recevoir un signal radio transmis par l'unité de transmission radio (16),

dans lequel le générateur de champ magnétique constant (25) est disposé dans le dispositif pouvant être inséré dans le corps (2) de telle sorte qu'une direction du champ magnétique constant est fixée, et

chacun des détecteurs de champ magnétique (6a-6h) est adapté pour détecter une direction du champ magnétique constant généré par le générateur de champ magnétique constant (25), et

le détecteur d'état de déplacement (3) comprend en outre un détecteur de direction d'orientation (41) qui est adapté pour détecter une direction d'orientation du dispositif dans le corps du sujet (1) en se basant sur la direction détectée par chacun des détecteurs de champ magnétique (6a-6h), et

le processeur d'état de déplacement (32) est adapté pour déterminer si une variation de position du dispositif pouvant être inséré dans le corps (2) est inférieure à un premier seuil prédéterminé, en se basant sur la position calculée par le calculateur de position (39), et est adapté pour déterminer si une variation de direction d'orientation du dispositif pouvant être inséré dans le corps (2) est inférieure à un second seuil prédéterminé, en se basant sur la direction d'orientation détectée par le détecteur de direction d'orientation (41), et

si la variation de position est inférieure au premier seuil prédéterminé, et la variation de direction d'orientation est inférieure au second seuil prédéterminé, alors le processeur d'état de déplacement (32) est adapté pour déterminer que le dispositif pouvant être inséré dans le corps (2) soit dans un état arrêté, et est adapté pour transmettre les informations indiquant que le dispositif pouvant être inséré dans le corps (2) est dans l'état arrêté, au dispositif pouvant être inséré dans le corps (2) pour que l'unité d'éclairage (11) et l'unité de collecte d'image (13) dans le dispositif pouvant être inséré dans le corps (2) arrêtent l'opération.

2. Système selon la revendication 1, dans lequel le détecteur d'état de déplacement (3) comprend en outre
une base de données de direction d'orientation (40) qui conserve, à l'avance, une distance du générateur de champ magnétique constant (25), une relation entre une direction du champ magnétique constant, et une direction d'orientation du dispositif dans le corps du sujet (1), et
le détecteur de direction d'orientation (41) est adapté pour détecter la direction d'orientation du dispositif dans le corps du sujet (1) en utilisant la base de données de direction d'orientation (40).

3. Système selon la revendication 1, dans lequel le détecteur d'état de déplacement (3) est adapté pour générer des informations d'état de déplacement pour commander un état d'entraînement de l'unité d'exécution de fonction (15) contenue dans le dispositif pouvant être inséré dans le corps (2) en se basant sur l'état de déplacement du dispositif pouvant être inséré dans le corps (2) dans le corps du sujet déterminé par le processeur d'état de déplacement (32).

# FIG.1

CAPSULE ENDOSCOPE
(BODY-INSERTABLE DEVICE)

SUBJECT
—1

TRAVEL STATE DETECTOR 3

FIXING MEMBER
7a

MAGNETIC FIELD DETECTOR
6a

RECEIVING ANTENNA
9

TRANSMITTING ANTENNA
10

TRAVEL STATE PROCESSOR
8

DISPLAY
4

PORTABLE RECORDING MEDIUM
5

EP 1 698 264 B1

EP 1 698 264 B1

# FIG.2

2

**CAPSULE ENDOSCOPE**

FUNCTION EXECUTING UNIT

15

11 — LED (ILLUMINATING UNIT)

12 — LED DRIVING CIRCUIT

13 — CCD (IMAGING UNIT)

14 — CCD DRIVING CIRCUIT

TRANSMITTING ANTENNA
17

16 — RF TRANSMITTING UNIT (RADIO UNIT)

PERMANENT MAGNET
25

N | S

18 — SYSTEM CONTROL CIRCUIT

24 — TRAVEL STATE INFORMATION DETECTING CIRCUIT

19 — RECEIVING ANTENNA

20 — SEPARATING CIRCUIT

21 — POWER REPRODUCING CIRCUIT

22 — BOOSTER CIRCUIT

23 — CAPACITOR

19

FIG.3

# FIG.4

```
         ┌──────────┐
         │  START   │
         └──────────┘
              │
              ▼
   ┌───────────────────────────┐
   │ SELECT MAGNETIC FIELD DETECTOR │  ~ S101
   │    AS REFERENCE DEVICE         │
   └───────────────────────────┘
              │
              ▼
   ┌───────────────────────────┐
   │ SELECT MAGNETIC FIELD DETECTORE AS │  ~ S102
   │   SELECTED DEVICE BASED ON         │
   │      REFERENCE DEVICE              │
   └───────────────────────────┘
              │
              ▼
   ┌───────────────────────────┐
   │ ACQUIRE MAGNETIC FIELD INTENSITY │  ~ S103
   │ RECEIVED BY REFERENCE DEVICE AND │
   │        SELECTED DEVICES          │
   └───────────────────────────┘
              │
              ▼
   ┌───────────────────────────┐
   │    CALCULATE DISTANCE     │  ~ S104
   └───────────────────────────┘
              │
              ▼
   ┌───────────────────────────┐
   │ CALCULATE POSITION OF CAPSULE │  ~ S105
   │   ENDOSCOPE BASED ON DISTANCE │
   └───────────────────────────┘
              │
              ▼
   ┌───────────────────────────┐
   │ OUTPUT POSITION OF CAPSULE │  ~ S106
   │  ENDOSCOPE TO TRAVEL STATE │
   │   INFORMATION GENERATOR    │
   └───────────────────────────┘
              │
              ▼
         ┌──────────┐
         │   END    │
         └──────────┘
```

# FIG.5

# FIG.6

START

INPUT POSITION OF CAPSULE ENDOSCOPE
AND MAGNETIC FIELD DIRECTION RECEIVED
BY SELECTED MAGNETIC FIELD
INTENSITY/MAGNETIC FIELD DIRECTION
DETECTOR — S201

CALCULATE RELATIVE POSITION OF
SELECTED DEVICES WITH RESPECT
TO CAPSULE ENDOSCOPE — S202

INPUT RELATIVE POSITION AND
MAGNETIC FIELD DIRECTION TO
ORIENTATION DIRECTION DATABASE,
AND DERIVE ORIENTATION DIRECTION — S203

OUTPUT ORIENTATION DIRECTION OF
CAPSULE ENDOSCOPE TO TRAVEL STATE — S204
INFORMATION GENERATOR

END

# FIG.7

MAGNETIC FIELD DETECTOR
6

$(a_1, a_2, a_3)$

$(x_1, y_1, z_1)$

CAPSULE
ENDOSCOPE
2

$(x, y, z)$

# FIG.8

```
          ┌──────────────┐
          │    START     │
          └──────┬───────┘
                 ▼
  ┌─────────────────────────────────┐
  │  INPUT POSITION AND ORIENTATION  │ ── S301
  │  DIRECTION OF CAPSULE ENDOSCOPE  │
  └─────────────────┬───────────────┘
                    ▼
  ┌─────────────────────────────────┐
  │  EXTRACT PAST DATA OF POSITION AND │ ── S302
  │      ORIENTATION DIRECTION         │
  └─────────────────┬───────────────┘
                    ▼
                          S303
                  IS
              POSITION
        CHANGE QUANTITY LESS THAN
              THRESHOLD
                  ?
```

No

S304

DERIVE TRAVEL SPEED

Yes

S305

IS CHANGE QUANTITY IN ORIENTATION DIRECTION LESS THAN THRESHOLD ?

Yes

No

S306

DETERMINE ENDOSCOPE TO BE IN STOP STATE

S307

DETERMINE ENDOSCOPE TO BE IN ORIENTATION DIRECTION VARIATION STATE

END

# FIG.9

TEST CAPSULE
43

CASING
45

S    N

46
PERMANENT MAGNET

47
FILLING MAMBER

# FIG.10

EP 1 698 264 B1

**EP 1 698 264 B1**

## REFERENCES CITED IN THE DESCRIPTION

### Patent documents cited in the description

- JP 2003019111 A
- US 20030114742 A1
- WO 0027281 A
- WO 200319111 A